# EUROPEAN PATENT SPECIFICATION

(11) **EP 2 316 453 B1**
(45) Date of publication and mention of the grant of the patent: **06.01.2016**
(21) Application number: 10075728.5
(22) Date of filing: 29.11.2006
(51) Int. Cl.: A61K 31/4412, A61P 43/00

(54) **Reduction of dizziness, a side effect associated with pirfenidone therapy**
Verringerung von Schwindel, einer Nebenwirkung im Zusammenhang mit einer Pirfenidon-Therapie
Réduction des vertiges, effets indésirables associés à une thérapie par la pirfénidone

(30) Priority: 02.12.2005 US 741976 P
(43) Date of publication of application: 04.05.2011
(62) Divisional of application: 06838621.8
(73) Proprietor: Intermune, Inc., Brisbane, CA 94005 (US)
(72) Inventor: Robinson, Cynthia Y., Burlingame California 94010 (US); Loutit, Jeffery Stuart, Los Altos California 94024 (US); Freemer, Michelle M., Mill Valley California 94941 (US)
(74) Representative: Potter Clarkson LLP

(56) References cited:
- EP-A1- 1 356 816
- WO-A2-2004/110245
- US-A- 5 310 562
- RAGHU G ET AL: "TREATMENT OF IDIOPATHIC PULMONARY FIBROSIS WITH A NEW ANTIFIBROTIC AGENT, PIRFENIDONE. RESULTS OF A PROSPECTIVE, OPEN-LABEL PHASE II STUDY", AMERICAN JOURNAL OF RESPIRATORY AND CRITICAL CARE MEDICINE, AMERICAN LUNG ASSOCIATION, NEW YORK, NY, US, vol. 159, no. 4, April 1999 (1999-04), pages 1061-1069, XP000878900, ISSN: 1073-449X
- LASKY J: "Pirfenidone: InterMune/Shionogi/Marnac", IDRUGS 2004 UNITED KINGDOM, vol. 7, no. 2, 2004, pages 166-172, XP002424042, ISSN: 1369-7056

## Description

### BACKGROUND OF THE INVENTION

### Field of the Invention

The invention relates to pirfenidone for use in decreasing adverse events associated with pirfenidone (5-methyl-1-phenyl-2-(1H)-pyridone) therapy.

### Description of the Related Art

Pirfenidone is small drug molecule whose chemical name is 5-methyl-1-phenyl-2-(1H)-pyridone. It is a non-peptide synthetic molecule with a molecular weight of 185.23 daltons. Its chemical elements are expressed as C₁₂H₁₁NO, and its structure and synthesis are known. Pirfenidone is manufactured commercially and being evaluated clinically as a broad-spectrum anti-fibrotic drug. Pirfenidone has anti-fibrotic properties via: decreased TGF-β expression, decreased TNF-α expression, decreased PDGF expression, and decreased collagen expression. Several pirfenidone Investigational New Drug Applications (INDs) are currently on file with the U.S. Food and Drug Administration. Phase II human investigations are ongoing or have recently been completed for pulmonary fibrosis, renal glomerulosclerosis, and liver cirrhosis. There have been other Phase II studies that used pirfenidone to treat benign prostate hypertrophy, hypertrophic scarring (keloids), and rheumatoid arthritis.

Pirfenidone is being investigated for therapeutic benefits to patients suffering from fibrosis conditions such as Hermansky-Pudlak Syndrome (HPS) associated pulmonary fibrosis and idiopathic pulmonary fibrosis (IPF). Pirfenidone is also being investigated for a pharmacologic ability to prevent or remove excessive scar tissue found in fibrosis associated with injured tissues including that of lungs, skin, joints, kidneys, prostate glands, and livers. Published and unpublished basic and clinical research suggests that pirfenidone may safely slow or inhibit the progressive enlargement of fibrotic lesions, and prevent formation of new fibrotic lesions following tissue injuries.

It is understood that one mechanism by which pirfenidone exerts its therapeutic effects is modulating cytokine actions. Pirfenidone is a potent inhibitor of fibrogenic cytokines and TNF-α. It is well documented that pirfenidone inhibits excessive biosynthesis or release of various fibrogenic cytokines such as TGF-β1, bFGF, PDGF, and EGF. Zhang S et al., Australian and New England J Ophthalmology 26:S74-S76 (1998). Experimental reports also show that pirfenidone blocks the synthesis and release of excessive amounts of TNF-α from macrophages and other cells. Cain et al., Int'l J Immunopharmacology 20:685-695 (1998).

As an investigational drug, pirfenidone is provided in tablet and capsule forms principally for oral administration. Various formulations have been tested and adopted in clinical trials and other research and experiments. The most common adverse reactions or events associated with pirfenidone therapy include gastrointestinal upset, nausea, fatigue, somnolence, dizziness, headache, and photosensitivity rash. Many of these effects can interfere with everyday activities and quality of life. These effects appear to be dose related. The adverse reactions associated with pirfenidone therapy are exacerbated when pirfenidone is administered at these higher doses.

Currently, adverse events following administration of pirfenidone are alleviated by dose reduction or discontinuation of pirfenidone. In a recent study, for adverse events rated Grade 2 or worse, the dosage was reduced in a stepwise manner: from 9 tablets per day to 6 tablets per day and 6 tablets per day to 3 tablets per day. Azuma, A. et al., Am J Respir Crit Care Med 171:1040-47 (2005). If, after a period of 14 days of observation with reduced dosage, the adverse event persisted or increased, the dosage was further reduced by one more step-from 6 tablets per day to 3 tablets per day. If the adverse event persisted or increased despite reducing the dosage to 3 tablets per day, the study medication was discontinued.

Raghu et al (1999) Am J Resp Crit Care Med 159(4), 1061-1069 describes a prospective open-label phase II study of pirfenidone in patients with IPF.

There remains an unmet clinical need for a method of administering higher doses of pirfenidone to a patient in a manner that eliminates or minimizes adverse events, such as nausea, vomiting, gastrointestinal upset, drowsiness, dizziness, headache, somnolence, and other potentially dangerous side effects that can occur with pirfenidone therapy.

### SUMMARY OF THE INVENTION

The invention disclosed herein is based on the unexpected finding that the administration of pirfenidone at or around the time food is consumed decreases the adverse events associated with the oral dosage form in humans. The invention is as described in the claims.

In an embodiment, the pirfenidone is in the form of a pharmaceutical composition. The uses involve, for example, administering a therapeutically effective amount of pirfenidone to a patient with food.

We disclose uses for reducing the likelihood of somnolence in a patient receiving pirfenidone therapy wherein the pirfenidone is in the form of a pharmaceutical composition. This may comprise, for example, administering a therapeutically effective amount of pirfenidone to the patient with food.

We disclose uses for reducing the likelihood of nausea in a patient receiving pirfenidone therapy wherein the pirfenidone is in the form of a pharmaceutical composition. The use comprises, for example, administering a therapeutically effective amount of pirfenidone to the patient with food.

In some embodiments the uses are for reducing the likelihood of headaches in a patient receiving pirfenidone therapy wherein the pirfenidone is in the form of a pharmaceutical composition. The use comprises, for example, administering a therapeutically effective amount of pirfenidone to the patient with food.

In some embodiments, the likelihood of one or more adverse effects is reduced. For example, in some embodiments, the likelihood of nausea and somnolence is reduced. In other embodiments, the likelihood of nausea and headaches is reduced. In still other embodiments, the likelihood of somnolence and headaches is reduced. In some embodiments, the likelihood of nausea, somnolence and headaches is reduced.

In some embodiments the uses are for administering pirfenidone to a patient, wherein the administering comprises providing pirfenidone in about 100 milligrams to about 400 milligrams per unit dosage form. The uses comprise providing one or more unit dosage forms three times per day to the patient. In an embodiment, the administering comprises providing one or more capsules comprising pirfenidone three times per day to the patient. In an embodiment, the administering comprises providing one or more capsules comprising about 267 mg of pirfenidone three times per day to the patient.

The administering comprises providing greater than 1800 mg/day to 4000 mg/day of pirfenidone to the patient. In some embodiments, the administering comprises providing from about 2200 mg/day to 4000 mg/day of pirfenidone to the patient. In some embodiments, the administering comprises providing from about 2400 mg/day to 4000 mg/day of pirfenidone to the patient. In an embodiment, the administering comprises providing about 2403 mg/day of pirfenidone to the patient.

In some embodiments, the food is a solid food with sufficient caloric and fat content that it is not rapidly dissolved and absorbed in the stomach. Thus, in some embodiments, the food is a meal, for example, breakfast, lunch or dinner.

In some embodiments, the therapeutically effective amount of pirfenidone is administered to the patient between about 1 hour prior to about 2 hours after eating a meal. In some embodiments, the pirfenidone is administered to the patient within about 30 minutes, about 15 minutes of consuming food.

The uses disclosed herein may further comprise providing information to prescribing physicians and patients receiving pirfenidone therapy useful for decreasing adverse events when taking pirfenidone. The uses may further comprise advising a patient to take pirfenidone with food. The uses may further comprise advising a patient to take pirfenidone with food to avoid and/or minimize adverse events associated with pirfenidone therapy.

The pirfenidone composition may be provided to the patient in a container associated with printed labeling advising that the administration with food results in a reduction in the likelihood of adverse events. The pirfenidone pharmaceutical composition may be provided to the patient in a container associated with printed labeling advising the patient that the pharmaceutical composition is to be administered at substantially the same time as consuming food.

Described herein is an article of manufacture or a kit comprising a container, wherein the container holds a pharmaceutical composition comprising pirfenidone in unit dosage form, and printed labeling instructions advising of the varying side effects when the composition is taken with and without food. The printed instructions may advise the patient to take the composition with food if stomach upset or somnolence occurs.

The printed instructions may further advise the patient that the administration of the composition with food results in a reduction in the likelihood of adverse events. The printed instructions may advise the patient to take the composition at substantially the same time as consuming food. The printed instructions may advise the patient to take the composition immediately after the consumption of food up to 1 hour after said consumption. The printed instructions may advise the patient to take the composition with a meal.

The printed instructions may advise the patient to take one or more of the capsules three times per day.

Also disclosed is pirfenidone for providing pirfenidone therapy to a patient, comprising providing a therapeutic dose of pirfenidone to the patient, and advising the patient that consuming the pirfenidone with food may reduce the incidence of adverse events resulting from pirfenidone therapy.

Also disclosed is pirfenidone for providing pirfenidone therapy to a patient, comprising providing a therapeutic dose of pirfenidone to the patient; and advising the patient that consuming the pirfenidone with food reduces mean maximum plasma concentration of pirfenidone.

The patient may be advised that consuming 801 mg pirfenidone with food reduces mean maximum plasma concentration of pirfenidone from 15724 ng/mL to 7874 ng/mL in comparison to consuming the pirfenidone without food. In addition, the patient may be advised that consuming 801 mg pirfenidone with food increases mean absorption half life of the pirfenidone from 0.572 hours to 1.78 hours in comparison to consuming the pirfenidone without food. It is contemplated that the patient may be advised in writing or orally, and that the written information may be contained (for example) in a label, a sticker, a product insert, product information, or prescribing information.

### BRIEF DESCRIPTION OF THE DRAWING

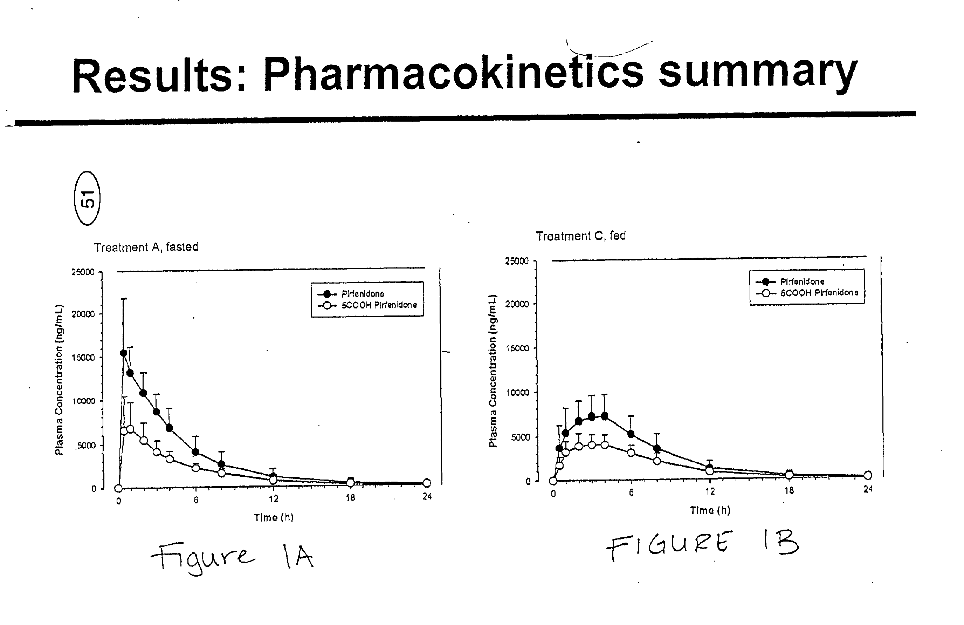

Figures 1A and 1B are graphs summarizing pharmacokinetic data for fasted and fed patients.

### DETAILED DESCRIPTION OF THE PREFERRED EMBODIMENT

The most common adverse reactions or events reported during pirfenidone therapy include gastrointestinal upset, nausea, fatigue, somnolence, dizziness, headache, and photosensitivity rash. Many of these effects can interfere with everyday activities and quality of life. These effects appear to be dose related, and are typically alleviated following dose-reduction or discontinuation of pirfenidone therapy. It is a novel discovery that administration of pirfenidone at or around the time food is consumed alleviates adverse events associated with the oral dosage form of pirfenidone in humans.

As used herein, the terms "adverse event" and "adverse reactions" refer to any unfavorable, harmful, or pathologic change in a patient receiving pirfenidone therapy as indicated by physical signs, symptoms, and/or clinically significant laboratory abnormalities that occur in a patient during the treatment and post-treatment period, regardless of suspected cause. This definition includes the following: intercurrent illness; injuries; exacerbation of pre-existing conditions; adverse events occurring as a result of product withdrawal, abuse, or overdose; and a change in a laboratory variable if considered by the attending physician to be clinically significant or if it caused (or should have caused) the clinician to reduce or discontinue the use of the product or initiate a non- protocol therapy or procedure.

The term "pirfenidone" includes salts thereof.

As used herein, the term "with food" is defined to mean, in general, the condition of having consumed food during the period between from about 1 hour prior to the administration of pirfenidone to about 2 hours after the administration of pirfenidone. In some embodiments, the food is a solid food with sufficient bulk and fat content that it is not rapidly dissolved and absorbed in the stomach. Preferably, the food is a meal, such as breakfast, lunch, or dinner. In some embodiments, the food is at least about 100 calories, about 200 calories, about 250 calories, about 300 calories, about 400 calories, about 500 calories, about 600 calories, about 700 calories, about 800 calories, about 900 calories, about 1000 calories, about 1250 calories, about 1500 calories.

The terms "without food," "fasted," or "on an empty stomach" are defined to mean the condition of not having consumed food within the time period of about 1 hour prior to the administration of pirfenidone to about 2 hours after the administration of pirfenidone. In some embodiments, food has not been consumed for about 10 hours, about 8 hours, about 6 hours, about 4 hours, about 2 hours prior to administration of pirfenidone.

The terms "patient" or "subject" refers to a human patient.

The uses disclosed herein include administering pirfenidone to a patient with food. The pirfenidone can be administered any time of day with food. For example, in some embodiments, the food can be consumed at any time during the period between from about 2 hours prior to the administration of pirfenidone to about 2 hours after the administration of pirfenidone. In some embodiments, the food can be consumed within the time period of about 2 hours, about 1.5 hours, about 1 hour, about 45 minutes, about 30 minutes, about 15 minutes, about 10 minutes, or about 5 minutes prior to the administration of pirfenidone. In some embodiments, the food can be consumed within the time period of about 5 minutes, about 10 minutes, about 15 minutes, about 30 minutes, about 45 minutes, about 1 hour, about 1.5 hours, or about 2 hours after the administration of pirfenidone. In some embodiments, the administration of pirfenidone to the patient is immediately after the consumption of food (e.g., within about 1 minute after food consumption) up to about 1 hour after food consumption. In some embodiments, pirfenidone is administered at substantially the same time as the consumption of the food.

According to the invention the effective daily intake of pirfenidone is greater than 1800 mg/dayto 4000 mg per day. In some embodiments, an effective daily intake of pirfenidone is between about 2400 and about 3600 mg per day. In some embodiments, an effective daily intake of pirfenidone is about 2403 mg/day.

In an embodiment, pirfenidone is administered to the subject in a unit dosage form comprising about 100 to about 400 mg of pirfenidone per unit. In an embodiment, pirfenidone is administered to the subject in a unit dosage form comprising about 267 mg of pirfenidone per capsule. In preferred embodiments, the unit dosage form is a capsule.

The dosing is three times daily, with one or more units per dose. In some embodiments, each dose comprises one, two, three or more unit dosage forms. In some embodiments, one or more units are administered to the subject three times per day. In some embodiments, 3 units are administered three times per day. In some embodiments, pirfenidone is administered as multiple doses spaced throughout the day and each dose comprises a therapeutically effective amount of pirfenidone.

As used herein, the term "unit dosage form," refers to physically discrete units suitable as unitary dosages for human and animal subjects, each unit containing a predetermined quantity of pirfenidone calculated in an amount sufficient to produce the desired effect in association with a pharmaceutically acceptable diluent, carrier or vehicle. In some embodiments, the unit dosage form is, for example, a pill, capsule, or tablet. In some embodiments, the unit dosage form is a capsule. In some embodiments, the amount of pirfenidone in a unit dosage form is about 100 mg to about 1800 mg, or about 200 mg to about 900 mg, or about 100 mg to about 400 mg. In an embodiment, the unit dosage form comprises about 267 mg of pirfenidone and is in the form of a capsule. In some embodiments, two or three capsules, each of which comprises about 267 mg of pirfenidone, are administered to the patient three times per day (e.g., up to a total daily intake of 2403 mg/day).

The uses are for administering a therapeutically acceptable amount of pirfenidone. The terms "therapeutically effective amount" and "prophylactically effective amount," as used herein, refer to an amount of pirfenidone sufficient to treat, ameliorate, or prevent the identified disease or condition, or to exhibit a detectable therapeutic, prophylactic, or inhibitory effect. The effect may be detected by any means known in the art. In some embodiments, the precise effective amount for a subject can depend upon the subject's body weight, size, and health; the nature and extent of the condition; and the therapeutic or combination of therapeutics selected for administration. Therapeutically and prophylactically effective amounts for a given situation may be determined by routine experimentation that is within the skill and judgment of the clinician.

The therapeutically or prophylactically effective amount of pirfenidone may be estimated initially either in cell culture assays or in animal models, usually rats, mice, rabbits, dogs, or pigs. The animal model may also be used to determine the appropriate concentration range and route of administration. Such information may then be used to determine useful doses and routes for administration in humans.

Therapeutic/prophylactic efficacy and toxicity may be determined by standard pharmaceutical procedures in cell cultures or experimental animals, e.g., ED₅₀ (the dose therapeutically effective in 50% of the population) and LD₅₀ (the dose lethal to 50% of the population). The dose ratio between therapeutic and toxic effects is the therapeutic index, and it may be expressed as the ratio, ED₅₀/LD₅₀. Pharmaceutical compositions that exhibit large therapeutic indices are preferred. However, the pharmaceutical compositions that exhibit narrow therapeutic indices are also within the scope of the embodiments. The data obtained from cell culture assays and animal studies may be used in formulating a range of dosage for human use. The dosage contained in such compositions is preferably within a range of circulating concentrations that include an ED₅₀ with little or no toxicity. The dosage may vary within this range depending upon the dosage form employed, sensitivity of the patient, and the route of administration.

More specifically, the maximum plasma concentrations (Cₘₐₓ) of pirfenidone may range from about 65 µM to about 115 µM, or about 75 µM to about 105 µM, or about 85 µM to about 95 µM, or about 85 µM to about 90 µM depending upon the route of administration. In general the daily intake will be in the range of about 100 mg/day to about 10 g/day, or about 200 mg to about 5 g/day, or about 400 mg to about 3 g/day, or about 500 mg to about 2 g/day, in single, divided, or continuous doses for a patient weighing between about 40 to about 100 kg (which doses may be adjusted for patients above or below this weight range, particularly children under 40 kg). Generally the daily intake will be in the range of about 25 mg/kg to about 200 mg/kg of body weight per day. The maximum daily intake of pirfenidone is 4 g/day.

The exact dosage will typically be determined by the practitioner, in light of factors related to the subject that requires treatment. Dosage and administration are generally adjusted to provide sufficient levels of pirfenidone or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half- life and clearance rate of the particular formulation.

The specifications for the unit dosage forms described herein depend on the particular dose employed and the effect to be achieved, and the pharmacodynamics associated with pirfenidone in the host.

The decrease in duration or number of adverse events in a patient receiving pirfenidone therapy can be evidenced in any suitable manner. Desirably, the oral administration of pirfenidone with food results in a reduction in the frequency and/or severity of adverse events as evidenced by a review of adverse events following administration of pirfenidone as compared to the administration of pirfenidone without food.

In some embodiments, pirfenidone is provided to a patient in a container associated with prescribing information that advises the patient to take the pharmaceutical composition with food, and in some embodiments further advises the patient that taking the composition with food results in a reduction in the duration, likelihood, and/or severity of adverse events associated with pirfenidone therapy. In some embodiments, the prescribing information advises the patient to take the composition with food if stomach upset and/or somnolence occurs.

The uses can include identifying a subject at risk for or suffering from an adverse event associated with pirfenidone therapy and administering a therapeutically effective amount of pirfenidone with food. The term "at risk for or suffering from" as used herein, refers to subjects having previously experienced, or currently experiencing, or having a high probability of experiencing an adverse event associated with pirfenidone therapy. Methods for identifying a subject at risk for or suffering from such adverse events are known in the art.

Disclosed uses include identifying a patient who could benefit from the uses disclosed herein. IUses described herein include identifying a subject who has experienced or is experiencing an adverse event, such as gastrointestinal symptoms, somnolence, and/or headache, following administration of pirfenidone. Identifying such subjects may be accomplished by any means that indicates a subject who may benefit from the methods disclosed herein, for example, by clinical diagnosis, laboratory testing, or any other means known to one of skill in the art, including any combination of means for identification.

It will be appreciated that the uses described herein include preventing, alleviating, and/or minimizing the duration and/or severity of adverse events associated with pirfenidone therapy.

The uses disclosed herein may result in a reduction in the likelihood of nausea in patients receiving pirfenidone therapy with food (fed) as compared to patients receiving pirfenidone therapy without food (fasted). Preferably, the likelihood of nausea of a fed population is reduced by at least about 25% relative to the likelihood of nausea of a fasted population; more preferably, the likelihood of nausea is reduced by at least about 30%; more preferably, reduced by at least about 33%; more preferably, reduced by at least about 40%; more preferably, reduced by at least about 50%; more preferably, reduced by at least about 60%; even more preferably, reduced by at least 70%; and most preferably, reduced by at least about 75%. Likelihood of nausea may be measured by any reproducible means of measurement.

The uses disclosed herein may result in a reduction in the likelihood of somnolence in patients receiving pirfenidone therapy with food (fed) as compared to patients receiving pirfenidone therapy without food (fasted). Preferably, the likelihood of somnolence of a fed population is reduced by at least about 25% relative to the likelihood of somnolence of a fasted population; more preferably, the likelihood of somnolence is reduced by at least about 30%; more preferably, reduced by at least about 33%; more preferably, reduced by at least about 40%; more preferably, reduced by at least about 50%; more preferably, reduced by at least about 60%; even more preferably, reduced by at least 70%; and most preferably, reduced by at least about 75%. Likelihood of somnolence may be measured by any reproducible means of measurement.

The uses disclosed herein may result in a reduction in the likelihood of headache in patients receiving pirfenidone therapy with food (fed) as compared to patients receiving pirfenidone therapy without food (fasted). Preferably, the likelihood of headache of a fed population is reduced by at least about 25% relative to the likelihood of headache of a fasted population; more preferably, the likelihood of headache is reduced by at least about 30%; more preferably, reduced by at least about 33%; more preferably, reduced by at least about 40%; more preferably, reduced by at least about 50%; more preferably, reduced by at least about 60%; even more preferably, reduced by at least 70%; and most preferably, reduced by at least about 75%. Likelihood of headache may be measured by any reproducible means of measurement.

The uses disclosed herein may result in a reduction in the likelihood of dizziness in patients receiving pirfenidone therapy with food (fed) as compared to patients receiving pirfenidone therapy without food (fasted). Preferably, the likelihood of dizziness of a fed population is reduced by at least about 25% relative to the likelihood of dizziness of a fasted population; more preferably, the likelihood of dizziness is reduced by at least about 30%; more preferably, reduced by at least about 33%; more preferably, reduced by at least about 40%; more preferably, reduced by at least about 50%; more preferably, reduced by at least about 60%; even more preferably, reduced by at least 70%; and most preferably, reduced by at least about 75%. Likelihood of dizziness may be measured by any reproducible means of measurement.

As described elsewhere herein, pirfenidone may be formulated in pharmaceutical compositions, if desired, and may be administered by any route that permits treatment of the disease or condition. A preferred route of administration is oral administration. Administration may take the form of single dose administration, or pirfenidone may be administered over a period of time, either in divided doses or in a continuous-release formulation or administration method (e.g., a pump). However pirfenidone is administered to the subject, the amount administered and the route of administration chosen should be selected to permit efficacious treatment of the disease condition.

### Pharmaceutical Compositions

While it is possible for pirfenidone to be administered alone, it may be preferable to formulate pirfenidone as pharmaceutical compositions. As such, pharmaceutical compositions useful in the uses of the invention are provided herein. More particularly, the pharmaceutical compositions described herein may be useful, *inter alia*, for treating or preventing neutropenia. A pharmaceutical composition is any composition that may be administered *in vitro* or *in vivo* or both to a subject in order to treat or ameliorate a condition. A pharmaceutical composition may be administered *in vivo*. A mammal includes any mammal, such as by way of non-limiting example, cattle, pigs, sheep, goats, horses, camels, buffalo, cats, dogs, rats, mice, and humans. A highly preferred subject mammal is a human.

The pharmaceutical compositions may be formulated with pharmaceutically acceptable excipients such as carriers, solvents, stabilizers, adjuvants, diluents, etc., depending upon the particular mode of administration and dosage form. The pharmaceutical compositions should generally be formulated to achieve a physiologically compatible pH, and may range from a pH of about 3 to a pH of about 11, preferably about pH 3 to about pH 7, depending on the formulation and route of administration. It may be preferred that the pH is adjusted to a range from about pH 5.0 to about pH 8. More particularly, the pharmaceutical compositions may comprise a therapeutically or prophylactically effective amount of pirfenidone, together with one or more pharmaceutically acceptable excipients. Optionally, the pharmaceutical compositions may comprise a combination of pirfenidone and a second active ingredient useful in the treatment or prevention of the disease or condition being treated.

Formulations, e.g., for parenteral or oral administration, are most typically solids, liquid solutions, emulsions or suspensions, while inhalable formulations for pulmonary administration are generally liquids or powders, with powder formulations being generally preferred. A preferred pharmaceutical composition may also be formulated as a lyophilized solid that is reconstituted with a physiologically compatible solvent prior to administration. Alternative pharmaceutical compositions may be formulated as syrups, creams, ointments, tablets, capsules and the like.

The term "pharmaceutically acceptable excipient" refers to an excipient for administration of a pharmaceutical agent, such as the compounds described herein. The term refers to any pharmaceutical excipient that may be administered without undue toxicity. Pharmaceutically acceptable excipients may include, for example, inactive ingredients such as disintegrators, binders, fillers, and lubricants used in formulating pharmaceutical products.

Pharmaceutically acceptable excipients are determined in part by the particular composition being administered, as well as by the particular method used to administer the composition. Accordingly, there exists a wide variety of suitable formulations of pharmaceutical compositions (see, e.g., Remington's Pharmaceutical Sciences).

Suitable excipients may be carrier molecules that include large, slowly metabolized macromolecules such as proteins, polysaccharides, polylactic acids, polyglycolic acids, polymeric amino acids, amino acid copolymers, and inactive virus particles. Other exemplary excipients include antioxidants such as ascorbic acid; chelating agents such as EDTA; carbohydrates such as dextrin, hydroxyalkylcellulose, hydroxyalkylmethylcellulose, stearic acid; liquids such as oils, water, saline, glycerol and ethanol; wetting or emulsifying agents; pH buffering substances; and the like. Liposomes are also included within the definition of pharmaceutically acceptable excipients.

Disintegrators include, for example, agar-agar, algins, calcium carbonate, carboxmethylcellulose, cellulose, clays, colloid silicon dioxide, croscarmellose sodium, crospovidone, gums, magnesium aluminium silicate, methylcellulose, polacrilin potassium, sodium alginate, low substituted hydroxypropylcellulose, and cross-linked polyvinylpyrrolidone hydroxypropylcellulose, sodium starch glycolate, and starch.

Binders include, for example, microcrystalline cellulose, hydroxymethyl cellulose, hydroxypropylcellulose, and polyvinylpyrrolidone.

Fillers include, for example, calcium carbonate, calcium phosphate, dibasic calcium phosphate, tribasic calcium sulfate, calcium carboxymethylcellulose, cellulose, dextrin derivatives, dextrin, dextrose, fructose, lactitol, lactose, magnesium carbonate, magnesium oxide, maltitol, maltodextrins, maltose, sorbitol, starch, sucrose, sugar, and xylitol.

Lubricants include, for example, agar, calcium stearate, ethyl oleate, ethyl laureate, glycerin, glyceryl palmitostearate, hydrogenated vegetable oil, magnesium oxide, magnesium stearate, mannitol, poloxamer, glycols, sodium benzoate, sodium lauryl sulfate, sodium stearyl, sorbitol, stearic acid, talc, and zinc stearate.

The pharmaceutical compositions described herein may be formulated in any form suitable for the intended method of administration. When intended for oral use for example, tablets, troches, lozenges, aqueous or oil suspensions, non-aqueous solutions, dispersible powders or granules (including micronized particles or nanoparticles), emulsions, hard or soft capsules, syrups or elixirs may be prepared. Compositions intended for oral use may be prepared according to any method known to the art for the manufacture of pharmaceutical compositions, and such compositions may contain one or more agents including sweetening agents, flavoring agents, coloring agents and preserving agents, in order to provide a palatable preparation.

Pharmaceutically acceptable excipients particularly suitable for use in conjunction with tablets include, for example, inert diluents, such as celluloses, calcium or sodium carbonate, lactose, calcium or sodium phosphate; disintegrating agents, such as cross-linked povidone, maize starch, or alginic acid; binding agents, such as povidone, starch, gelatin or acacia; and lubricating agents, such as .magnesium stearate, stearic acid or talc.

Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed. To those skilled in the pharmaceutical research and manufacturing, it is generally known that tablet formulations permit generous additions of inactive ingredients including excipients and coating substances, and a high percentage of fillers. However, the addition of inactive ingredients may limit the amount of active ingredients carried in each tablet.

Formulations for oral use may be also presented as hard gelatin capsules where the active ingredient is mixed with an inert solid diluent, for example celluloses, lactose, calcium phosphate or kaolin, or as soft gelatin capsules wherein the active ingredient is mixed with non-aqueous or oil medium, such as glycerin, propylene glycol, polyethylene glycol, peanut oil, liquid paraffin or olive oil. Capsules may allow for inclusion of a larger amount of binders, instead of fillers as used more in tablets. The capsule shell may be made of hard gelatin in an embodiment. The shell may be clear or opaque, white or with color in various embodiments. In an embodiment, the capsule is size 1. Other sizes may be adopted in alternative, embodiments. The benefits of using capsules include their slender shape, which make them easy to swallow and their ability to effectively mask unpleasant taste and/or odor associated with pirfenidone, resulting in higher patient satisfaction and greater patient compliance with pirfenidone therapy dosing regimens.

Pharmaceutical compositions may be formulated as suspensions comprising pirfenidone in admixture with at least one pharmaceutically acceptable excipient suitable for the manufacture of a suspension.

Pharmaceutical compositions may be formulated as dispersible powders and granules suitable for preparation of a suspension by the addition of suitable excipients.

Excipients suitable for use in connection with suspensions include suspending agents, such as sodium carboxymethylcellulose, metbylcellulose, hydroxypropyl methylcellulose, sodium alginate, polyvinylpyrrolidone, gum tragacanth, gum acacia, dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycethanol), a condensation product of ethylene oxide with a partial ester derived from a fatty acid and a hexitol anhydride (e.g., polyoxyethylene sorbitan monooleate); and thickening agents, such as carbomer, beeswax, hard paraffin or cetyl alcohol. The suspensions may also contain one or more preservatives such as acetic acid, methyl and/or n-propyl p-hydroxy-benzoate; one or more coloring agents; one or more flavoring agents; and one or more sweetening agents such as sucrose or saccharin.

The pharmaceutical compositions may also be in the form of oil-in water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth; naturally occurring phosphatides, such as soybean lecithin, esters or partial esters derived from fatty acids; hexitol anhydrides, such as sorbitan monooleate; and condensation products of these partial esters with ethylene oxide, such as polyoxyethylene sorbitan monooleate. The emulsion may also contain sweetening and flavoring agents. Syrups and elixirs may be formulated with sweetening agents, such as glycerol, sorbitol or sucrose. Such formulations may also contain a demulcent, a preservative, a flavoring or a coloring agent.

Additionally, the pharmaceutical compositions may be in the form of a sterile injectable preparation, such as a sterile injectable aqueous emulsion or oleaginous suspension. This emulsion or suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as a solution in 1,2-propane-diol.

The sterile injectable preparation may also be prepared as a lyophilized powder. Among the acceptable vehicles and solvents that may be employed are water, Ringer's solution, and isotonic sodium chloride solution. In addition, sterile fixed oils may be employed as a solvent or suspending medium. For this purpose any bland fixed oil may be employed including synthetic mono- or diglycerides. In addition, fatty acids such as oleic acid may likewise be used in the preparation of injectables.

To obtain a stable water-soluble dose form of a pharmaceutical composition, pirfenidone may be dissolved in an aqueous solution of an organic or inorganic acid, such as 0.3 M solution of succinic acid, or more preferably, citric acid. Pirfenidone may be dissolved in a suitable co-solvent or combination of co-solvents. Examples of suitable co-solvents include alcohol, propylene glycol, polyethylene glycol 300, polysorbate 80, glycerin and the like in concentrations ranging from about 0 to about 60% of the total volume. In an embodiment, pirfenidone is dissolved in DMSO and diluted with water.

The pharmaceutical composition may also be in the form of a solution of pirfenidone in an appropriate aqueous vehicle, such as water or isotonic saline or dextrose solution. Also included in the definition of pirfenidone are compounds which have been modified by substitutions or additions of chemical or biochemical moieties to pirfenidone which make them more suitable for delivery (e.g., increase solubility, bioactivity, palatability, decrease adverse reactions, etc.), for example by esterification, glycosylation, PEGylation, etc.

In a preferred embodiment, pirfenidone may be formulated for oral administration in a lipid-based formulation suitable for low solubility compounds. Lipid-based formulations may generally enhance the oral bioavailability of pirfenidone.

As such, a preferred pharmaceutical composition comprises a therapeutically or prophylactically effective amount of pirfenidone, together with at least one pharmaceutically acceptable excipient selected from the group consisting of medium chain fatty acids or propylene glycol esters thereof (e.g., propylene glycol esters of edible fatty acids such as caprylic and capric fatty acids) and pharmaceutically acceptable surfactants such as polyoxyl 40 hydrogenated castor oil.

In an alternative preferred embodiment, cyclodextrins may be added as aqueous solubility enhancers. Preferred cyclodextrins include hydroxypropyl, hydroxyethyl, glucosyl, maltosyl and maltotriosyl derivatives of [alpha]-, [beta]-, and [gamma]- cyclodextrin. A particularly preferred cyclodextrin solubility enhancer is hydroxypropyl- *o*-cyclodextrin (BPBC), which may be added to any of the above-described compositions to further improve the aqueous solubility characteristics of pirfenidone. In an embodiment, the composition comprises about 0.1% to about 20% hydroxypropyl-o- cyclodextrin, more preferably about 1% to about 15% hydroxypropyl-*o*-cyclodextrin, and even more preferably from about 2.5% to about 10% hydroxypropyl-[sigma]-cyclodextrin. The amount of solubility enhancer employed will depend on the amount of pirfenidone in the composition.

A pharmaceutical composition preferably contains a total amount of pirfenidone sufficient to achieve an intended therapeutic effect. The total amounts of pirfenidone that may be combined with the carrier materials to produce a unitary dosing form will vary depending upon the host treated and the particular mode of administration. Preferably, the compositions are formulated so that a daily intake of between 0.01 to 100 mg/kg body weight/day of pirfenidone is administered to a subject receiving the compositions.

In an embodiment, the composition is provided in the form of a capsule wherein by weight, 2-10% of the capsule is disintegrator, 2-30% is binder, 2-30% is filler, and 0.3-0.8% is lubricant. A multitude of substances may be suitably included as disintegrator, binder, filler, and lubricant. One example is to use magnesium stearate as lubricant, microcrystalline cellulose as binder, and croscarmellose as disintegrator. In an embodiment, the capsule formulation further includes povidone. By weight povidone may constitute 1-4% of the capsule. For example, in an embodiment of the invention, the composition is formulated as a capsule comprising 82.15% pirfenidone, 8.15% croscarmellose sodium, 7.38% microcrystalline cellulose, 1.85% povidine, USP, EP, and 0.46% magnesium stearate.

### EXAMPLES

### Reference EXAMPLE 1

### SINGLE-DOSE STUDY

A study was designed to evaluate the effect of food, antacids, and food taken with antacids on adverse events associated with pirfenidone use. The trial was conducted as a randomized, open-label, four-treatment crossover, with a single dose for each treatment period and a 2-day washout period between study treatments. 16 healthy adults between the ages of 50 and 79 years having body mass indices between 18 and 30 (inclusive) were enrolled and completed all 4 treatment arms. The treatment arms were as follows:
A) pirfenidone alone (Fasted);
B) pirfenidone within 1 minute following a dose of antacid (20 mL Mylanta® Maximum Strength Liquid) (Fasted + Antacid);
C) pirfenidone 5 minutes after completing a standard meal (Fed); and
D) pirfenidone 5 minutes after completing a standard meal, then within 1 minute, followed by a dose of antacid (Fed + Antacid).

All subjects were admitted to the clinic for clinical evaluation the day prior to receiving the first dose of the study medication and remained confined for 13 days. Following an overnight fast of at least 10 hours (h), subjects were randomized to one of the above-described 4 treatment sequences. Each dose of pirfenidone (3 x 267 mg capsules) was administered orally with 240 mL room temperature water on days 1, 4, 7, and 10 in the morning, following an overnight fast of at least 10 hours. The washout period between treatments was 2 days. A standard meal consisted of 2 fried eggs, 2 strips of bacon, 2 slices of toast, 2 pats of butter, hash brown potatoes (4 oz), and whole milk (8 oz).

On each study day, adverse events were reviewed with the patients as follows. Before administration of the first dose of the study drug, study site personnel noted the occurrence and nature of each subject's medical condition(s). During the trial, site personnel again noted any change in the condition(s) or occurrence and nature of any adverse events. The severity of adverse events were graded based on the Modified Common Toxicity Criteria and the relationship of the study drug to the adverse event determined.

Of subjects receiving pirfenidone therapy without food, 9 (or 56.3%) experienced an adverse event. Of subjects receiving pirfenidone therapy within 1 minute following a dose of antacid, 9 (56.3%) experienced an adverse event. In comparison, of subjects receiving pirfenidone therapy with food, 5 (31.3%) experienced an adverse event, and 4 (25%) subjects receiving pirfenidone therapy with food and antacid experienced an adverse event. In total, 12 subjects (75%) had at least 1 adverse event. In 8 of these 12 subjects (75%), the adverse events were mild. The most common adverse events were nausea (7 of 16 or 44%), dizziness (6 of 16 or 38%), and somnolence (4 of 16 or 25%).

Of 7 subjects who reported nausea, 5 of 16 (31%) were fasted compared to only 2 of 16 (13%) fed subjects. Of 5 subjects reporting dizziness, 4 of 16 (25%) were fasted, while only 1 of 16 (6%) was fed. Of 3 subjects reporting somnolence, 2 of 16 (13%) were fasted and 1 of 16 (6%) were fed. Of 3 subjects reporting headaches, 2 of 16 (13%) were fasted and 1 of 16 (6%) were fed. Pharmacokinetic data for fed and fasted patients are summarized in Figures 1A and 1B.

### Reference EXAMPLE 2

### MULTIPLE-DOSE STUDY

A second study was designed to examine incidences of adverse events on multiple ascending daily doses of pirfenidone. The trial was conducted as an open-label, escalating-dose study with no washout period between dose escalations. 25 healthy adults between the ages of 45 and 79 (inclusive) having body mass indices between 18 and 30 (inclusive) were enrolled. 22 adults completed the treatment. Each volunteer received from 801 mg/day to 4005 mg/day of pirfenidone divided into three equal doses as follows:
- Days 1-2:: 1 capsule three times a day (TID) (801 mg total daily dose (TDD))
- Day 3:: 1 capsule in the morning (~0800) (267 mg TDD)
- Days 4-5:: 2 capsules TID (1602 mg TDD)
- Day 6:: 2 capsules in the morning (534 mg TDD)
- Days 7-8:: 3 capsules TID (2403 mg TDD)
- Day 9:: 3 capsules in the morning (801 mg TDD)
- Days 10-11:: 4 capsules TID (3204 mg TDD)
- Day 12:: 4 capsules in the morning (1068 mg TDD)
- Days 13-14:: 5 capsules TID (4005 mg TDD)
- Day 15:: 5 capsules in the morning (1335 mg TDD)

All subjects were admitted to the clinic for clinical evaluation the day before the first dose of study medication and remained confined for 17 days. Each subject received all dose levels of the study drug unless adverse events or the clinical judgment of the trial physician precluded the continuation of treatment. All doses were taken with 240 mL room temperature water and with food (excluding grapefruit juice). TID doses times were ~0800, ~1200, and ~1800.

23 (92%) of all subjects had at least one adverse event. In 22 of these 23 subjects (96%), the adverse events were mild. The most common adverse events were nausea (9 of 25 or 36%), headache (7 of 25 or 28%), and somnolence (8 of 25 or 32%).

Also disclosed herein are uses according to the following numbered paragraphs.
1. Pirfenidone for use in a method of reducing adverse events in a patient receiving pirfenidone therapy comprising administering to the patient a pharmaceutical composition with food, wherein the composition comprises a therapeutically effective amount of pirfenidone, and wherein the administering comprises providing greater than 1800 mg/day of pirfenidone to the patient.
2. The pirfenidone for use of paragraph 1, wherein the patient is a human.
3. The pirfenidone for use of paragraphs 1 or 2, wherein the administering comprises orally administering the pharmaceutical composition.
4. The pirfenidone for use of any one of paragraphs 1-3, wherein the administering to the patient occurs between 30 minutes prior to and 2 hours after consuming food.
5. The pirfenidone for use of any one of paragraphs 1-4, wherein the administering to the patient is substantially at the same time as the consumption of the food.
6. The pirfenidone for use of any one paragraphs 1-5, wherein the administering to the patient is immediately after the consumption of food up to 1 hour after said consumption.
7. The pirfenidone for use of any one of paragraphs 1-6, wherein the administering with food comprises administering pirfenidone with a meal.
8. The pirfenidone for use of any one of paragraphs 1-7, wherein the administering comprises providing a unit dosage form comprising pirfenidone.
9. The pirfenidone for use of any one of paragraphs 1-8, wherein the administering comprises providing a tablet or capsule, wherein the tablet or capsule comprises pirfenidone.
10. The pirfenidone for use of any one of paragraphs1-9, wherein the administering comprises providing one or more of tablets or capsules comprising pirfenidone to the patient one or more times per day.
11. The pirfenidone for use of any one of paragraphs 1-10, wherein the administering comprises providing one or more of capsules comprising pirfenidone to the subject twice per day.
12. The pirfenidone for use of any one of paragraphs 1-11, wherein the administering comprises providing one or more capsules comprising pirfenidone to the subject three times per day.
13. The pirfenidone for use of any one of paragraphs 1-12, wherein the administering comprises providing a unit dosage form of pirfenidone, wherein the unit dosage form comprises about 100 to about 400 milligrams of pirfenidone per unit.
14. The pirfenidone for use of any one of paragraphs 1-13, wherein the administering comprises providing a unit dosage form of pirfenidone, wherein the unit dosage form comprises about 267 milligrams of pirfenidone per unit.
15. The pirfenidone for use of any one of paragraphs 1-14, wherein the administering comprises providing from about 2000 mg,/day to about 4000 mg/day of pirfenidone to the patient.
16. The pirfenidone for use of any one of paragraphs 1-15, wherein the administering comprises providing from about 2200 mg/day to about 4000 mg/day of pirfenidone to the patient.
17. The pirfenidone for use of any one of paragraphs 1-16, wherein the administering comprises providing from about 2400 mg/day to about 4000 mg/day of pirfenidone to the patient.
18. The pirfenidone for use of any one of paragraphs 1-17, wherein the administering comprises providing about 2403 mg/day of pirfenidone to the patient.
19. The pirfenidone for use of any one of paragraphs 1-18, further comprising advising the patient that the administration of the pharmaceutical composition with food results in a reduction in the likelihood of adverse events.
20. The pirfenidone for use of any one of paragraphs 1-19, wherein the administering comprises providing said pharmaceutical composition to the patient in a container associated with printed labeling advising the patient that the pharmaceutical composition is to be administered with food.
21. The pirfenidone for use of any one of paragraphs 1-20, wherein the adverse event is nausea.
22. The pirfenidone for use of any one of paragraphs 1-20, wherein the adverse event is somnolence.
23. The pirfenidone for use of any one of paragraphs 1-20, wherein the adverse event is headache.
24. A kit comprising a pharmaceutical composition, prescribing information, and a container, wherein the pharmaceutical composition comprises a therapeutically effective amount of pirfenidone.
25. The kit of paragraph 24, wherein the prescribing information advises a patient to take the pharmaceutical composition with food.
26. The kit of any paragraphs 24 or 25, wherein a tablet or capsule comprises the composition.
27. The kit of paragraph 26, wherein the prescribing information advises the patient to take one or more of the tablets or capsules one or more times per day.
28. The kit of any one of paragraphs 24-27, wherein the composition comprises a unit dosage form comprising from about 100 to about 400 milligrams of pirfenidone per unit.
29. Pirfenidone for use in a method for providing pirfenidone therapy to a patient, comprising: providing a therapeutic dose of pirfenidone to the patient; and advising the patient to take the pirfenidone with food.
30. Pirfenidone for use in a method for providing pirfenidone therapy to a patient, comprising: providing a therapeutic dose of pirfenidone to the patient; and advising the patient that consuming the pirfenidone with food may reduce the incidence of adverse events resulting from pirfenidone therapy.
31. Pirfenidone for use in a method for providing pirfenidone therapy to a patient, comprising: providing a therapeutic dose of pirfenidone to the patient; and advising the patient that consuming the pirfenidone with food reduces mean maximum plasma concentration of pirfenidone.
32. The pirfenidone for use of paragraph 31, further comprising advising the patient that consuming 801 mg pirfenidone with food reduces mean maximum plasma concentration of pirfenidone from 15724 ng/mL to 7874 ng/mL in comparison to consuming the pirfenidone without food.
33. The pirfenidone for use of paragraph 31, further comprising advising the patient that consuming 801 mg pirfenidone with food increases mean absorption half life of the pirfenidone from 0.572 hours to 1.78 hours in comparison to consuming the pirfenidone without food.
34. The pirfenidone for use of paragraph 31, wherein the patient is advised in writing.
35. The pirfenidone for use of paragraph 31, wherein the patient is advised orally.

## Claims

1. Pirfenidone, including salts thereof, for use in reducing dizziness in a patient receiving pirfenidone therapy, wherein the pirfenidone or salt is for administration with food, wherein the administration comprises providing to the subject greater than 1800 mg/day to 4000 mg/day, wherein one or more unit dosage forms are administered to the subject three times per day.

2. Use of pirfenidone or a salt thereof in the manufacture of a medicament for reducing dizziness in a patient receiving pirfenidone therapy, wherein the medicament is for administration with food, wherein the administration comprises providing to the subject greater than 1800 mg/day to 4000 mg/day, wherein one or more unit dosage forms are administered to the subject three times per day.

3. The pirfenidone, salt or use of any one of claims 1 to 2, wherein the pirfenidone, salt or medicament is for administration within 30, 15 or 5 minutes after consuming the food.

4. The pirfenidone, salt or use of any one of claims 1 to 2, wherein the pirfenidone, salt or medicament is for administration within 30, 15 or 5 minutes before consuming the food.

5. The pirfenidone, salt or use of any one of claims 1 to 2, wherein the pirfenidone, salt or medicament is for administration at the same time as consuming the food.

6. The pirfenidone, salt or use of any one of claims 1 to 5, wherein the pirfenidone, salt or medicament is for administration with a meal.

7. The pirfenidone, salt or use of any one of claims 1, 2, 5 or 6 wherein the unit dosage form is a pill, capsule, or tablet.

8. The pirfenidone, salt or use of any one of claim 1, 2, 5 or 6, wherein the unit dosage form comprises 267 mg of pirfenidone.

9. The pirfenidone, salt or use of any one of claims 1 to 6, wherein three unit dosage forms are administered three times per day.

10. The pirfenidone, salt or use of any one of claims 1 to 9, wherein the patient receiving pirfenidone therapy has idiopathic pulmonary fibrosis, pulmonary fibrosis, renal glomerulosclerosis, liver cirrhosis, benign prostate hypertrophy, hypertrophic scarring, rheumatoid arthritis, Hermansky-Pudlak Syndrome (HPS) associated pulmonary fibrosis, or is in need of prevention or removal of excessive scar tissue found in fibrosis associated with injured tissues including that of lungs, skin, joints, kidneys, prostate glands and livers, or is in need of an anti-fibrotic drug or in need of slowing or inhibiting of progressive enlargement of fibrotic lesions or prevention of formation of new fibrotic lesions following tissue injuries.

## Patentansprüche

1. Pirfenidon, einschlie•lich Salze davon, zur Verwendung bei der Verringerung von Schwindelgef,hl bei einem Patienten, der eine Pirfenidontherapie erh *f* lt, wobei das Pirfenidon oder das Salz davon f,r die Verabrei chung zusammen mit Nahrung gedacht ist, wobei die Verabreichung die Bereitstellung von mehr als 1800 mg/Tag bis 4000 mg/Tag Pirfenidon an das Subjekt umfasst, wobei eine oder mehrere Einheitsdosierungsformen dreimal am Tag an das Subjekt verabreicht werden.

2. Verwendung von Pirfenidon oder eines Salzes davon bei der Herstellung eines Medikamentes zur Verringerung von Schwindelgef,hl bei einem Patienten, welcher eine Pirfenidonthera - pie erhflt, wobei das Medikament f,r die Verabreichung zusammen mit Nahrung gedacht ist, wobei die Verabreichung die Bereitstellung von mehr als 1800 mg/Tag bis 4000 mg/Tag Pirfenidon an das Subjekt umfasst, wobei eine oder mehrere Einheitsdosierungsformen dreimal am Tag an das Subjekt verabreicht werden.

3. Pirfenidon, das Salz oder die Verwendung nach einem der Anspr,che 1 bis 2, wobei das Pirfenidon, das Salz oder das Medikament f,r die Verabreichung innerhalb 30, 15 oder 5 Minuten nach der Nahrungsaufnahme bestimmt ist.

4. Pirfenidon, das Salz oder die Verwendung nach einem der Anspr,che 1 bis 2, wobei das Pirfenidon, das Salz oder das Medikament f,r die Verabreichung innerhalb 30, 15 oder 5 Minuten vor dem Verzehr der Nahrung bestimmt ist.

5. Pirfenidon, das Salz oder die Verwendung nach einem der Anspr,che 1 bis 2, wobei das Pirfenidon, das Salz oder das Medikament f,r die Verabreichung zur selben Zeit wie der Verzehr der Nahrung bestimmt ist.

6. Pirfenidon, das Salz oder die Verwendung nach einem der Anspr,che 1 bis 5, wobei das Pirfenidon, das Salz oder das Medikament f,r die Verabreichung z usammen mit einer Mahlzeit bestimmt ist.

7. Pirfenidon, das Salz oder die Verwendung nach einem der Anspr, che 1, 2, 5 oder 6, wobei die Einheitsdosierungsform eine Pille, Kapsel oder Tablette ist.

8. Pirfenidon, das Salz oder die Verwendung nach einem der Anspr,che 1, 2, 5 oder 6, wobei die Einheitsdosierungsform 267 mg Pirfenidon umfasst.

9. Pirfenidon, das Salz oder die Verwendung nach einem der Anspr,che 1 bis 6, wobei dreimal am Tag drei Einheitsdosierungsformen verabreicht werden.

10. Pirfenidon, das Salz oder die Verwendung nach einem der Anspr,che 1 bis 9, wobei der die Pirfenidontherapie erhaltende Patient idiopathische Lungenfibrose, Lungenfibrose, Nieren-Glomerulosklerose, Leberzirrhose, gutartige Prostata Hypertrophie, hypertrophe Narbenbildung, rheumatische Arthritis, Hermansky-Pudlak Syndrom (HPS) verbunden mit Lungenfibrose hat oder wenn er die Prfvention oder Entfernung von exzessivem Narbengewebe ben"tigt, welches in einer Fibrose gefunden wird, die mit verletztem Gewebe einschlie•lich de m von Lungen, Haut, Gelenken, Nieren, Prostatadr,sen und Lebern verbunden ist, oder wenn er ein anti - fibrotisches Medikament ben"tigt oder die Verlangsamung oder Verhinderung einer progressiven Vergr"•erung fibrotischer Lfsionen oder Prfvention der Bildung neuer fibrotischer Lfsionen infolge von Gewebeverletzungen ben"tigt.

## Revendications

1. Pirfénidone, incluant les sels de celle-ci, pour une utilisation dans la réduction des vertiges chez un patient recevant une thérapie par pirfénidone, laquelle pirfénidone ou lequel sel est destiné(e) à l'administration avec un aliment, dans laquelle l'administration comprend la fourniture au sujet de plus de 1 800 mg/jour à 4 000 mg/jour, dans laquelle une ou plusieurs formes de dosage unitaires sont administrées au sujet trois fois par jour.

2. Utilisation de pirfénidone ou d'un sel de celle-ci dans la fabrication d'un médicament pour réduire des vertiges chez un patient recevant une thérapie par pirfénidone, dans laquelle le médicament est destiné à l'administration avec un aliment, dans laquelle l'administration comprend la fourniture au sujet de plus de 1 800 mg/jour à 4 000 mg/jour, dans laquelle une ou plusieurs formes de dosage unitaires sont administrées au sujet trois fois par jour.

3. Pirfénidone, sel ou utilisation selon l'une quelconque des revendications 1 à 2, dans laquelle (lequel) la pirfénidone, le sel ou le médicament est destiné(e) à une administration dans les 30, 15 ou 5 minutes après consommation de l'aliment.

4. Pirfénidone, sel ou utilisation selon l'une quelconque des revendications 1 à 2, dans laquelle (lequel) la pirfénidone, le sel ou le médicament est destiné (e) à une administration dans les 30, 15 ou 5 minutes avant consommation de l'aliment.

5. Pirfénidone, sel ou utilisation selon l'une quelconque des revendications 1 à 2, dans laquelle (lequel) la pirfénidone, le sel ou le médicament est destiné(e) à l'administration en même temps que la consommation de l'aliment.

6. Pirfénidone, sel ou utilisation selon l'une quelconque des revendications 1 à 5, dans laquelle (lequel) la pirfénidone, le sel ou le médicament est destiné(e) à l'administration avec un repas.

7. Pirfénidone, sel ou utilisation selon l'une quelconque des revendications 1, 2, 5 ou 6, dans laquelle (lequel) la forme de dosage unitaire est une pilule, une capsule ou un comprimé.

8. Pirfénidone, sel ou utilisation selon l'une quelconque des revendications 1, 2, 5 ou 6, dans laquelle (lequel) la forme de dosage unitaire comprend 267 mg de pirfénidone.

9. Pirfénidone, sel ou utilisation selon l'une quelconque des revendications 1 à 6, dans laquelle (lequel) trois formes de dosage unitaire sont administrées trois fois par jour.

10. Pirfénidone, sel ou utilisation selon l'une quelconque des revendications 1 à 9, dans laquelle (lequel) le patient recevant une thérapie par pirfénidone est atteint d'une fibrose pulmonaire idiopathique, d'une fibrose pulmonaire, d'une glomérulosclérose rénale, d'une cirrhose du foie, d'une hypertrophie bénigne de la prostate, d'une cicatrisation hypertrophique, d'une polyarthrite rhumatoïde, d'une fibrose pulmonaire associée au syndrome de Hermansky-Pudlak (SHP), ou est en besoin de prévention ou d'élimination d'un excès de tissu cicatriciel que l'on trouve dans une fibrose associée à des tissus blessés incluant ceux des poumons, de la peau, des articulations, des reins, des glandes de la prostate et du foie, ou est en besoin d'un médicament antifibrotique ou en besoin d'un ralentissement ou d'une inhibition d'un grossissement progressif de lésions fibreuses ou d'une prévention de la formation de nouvelles lésions fibreuses suite à des blessures tissulaires.
